# EUROPÄISCHE PATENTANMELDUNG

(11) **EP 1 510 196 A1**
(43) Veröffentlichungstag der Anmeldung: **02.03.2005**
(21) Anmeldenummer: 04015712.5
(22) Anmeldetag: 03.07.2004
(51) Int. Cl.: A61K 6/087

(54) **Zahnfüllungsmaterial**

(30) Priorität: 26.08.2003 DE 10339557
(71) Anmelder: Heraeus Kulzer GmbH, 63450 Hanau (DE)
(72) Erfinder: Brämer, Wulf, Dr., 63486 Bruchköbel (DE); Koops, Ulrich, Dr., 64380 Rossdorf (DE); Dehm, Gerhard, 63579 Freigericht (DE)
(74) Vertreter: Kühn, Hans-Christian

(57) **Zusammenfassung**

Es wird ein Zahnfüllungsmaterial aus Pulver und Flüssigkeit beschrieben, im wesentlichen bestehend aus
A einer organischen selbsthärtenden Komponente,
B einer anorganischen Komponente, die eine Abbindereaktion unter Kristallbildung und Expansion eingeht,
C Wasser,
mit der charakteristischen Eigenschaft, dass es nach Anmischen in einer Verarbeitungszeit von 2 bis 5 Minuten plastisch verformbar bleibt und dann binnen 10 bis 15 Minuten härtet.

## Beschreibung

Die Erfindung betrifft ein Zahnfüllungsmaterial.

In der Zahnmedizin kommt man immer mehr vom Einsatz von Amalgam als Zahnfüllungsmaterial ab, sowohl wegen des grau-schwarzen Farbeindrucks als auch wegen Bedenken hinsichtlich der Toxizität von Quecksilber.

Eine Alternative bilden die zahnfarbenen Composite-Füllungen, die schichtweise in die Kavität eingebracht und mit Licht gehärtet werden. Durch die Schrumpfung bei der Polymerisation ergeben sich aber Probleme dort, wo Zahn und Füllmaterial offenliegen. Die sogenannte Randspaltbildung führt zur erhöhten Gefahr weiteren Kariesbefalls.

Man hat z.B. schrumpfungsarme, unter Ringöffnung polymerisierende Polymere (ROMP, DE 199 05 093 A1) oder Polymere auf Siloxan-Basis (z.B. DE 41 33 494 C2) vorgeschlagen, um diesem Problem beizukommen. Die Tatsache, dass die herkömmlichen anorganischen Füller wie Gläser, Glaskeramik oder Silikatpulver ihre Dimension nicht verändern, führt aber dazu, dass insgesamt immer eine leichte Schrumpfung des Materials bleibt.

DE 38 13 607A1 beschreibt einen mit Metall gefüllten Composite-Werkstoff für die Zahnrestauration, der eine gering expandierend anorganische Matrix aufweist, insbesondere eine kristallisationsfähige anorganische Substanz. DE 100 21 605 A1 betrifft ein Zahnfüllmaterial, besonders eine Wurzelkanalfüllmasse enthaltend eine beim Aushärten durch chemische, physikochemische oder physikalische Effekte expandierende Komponente, besonders ein System aus Ammoniumsalz und basischem Calciumsalz. Die expandierende Komponente ist dabei in solchem Maße vorhanden, dass das gesamte Material beim Aushärten expandiert.

Ein weiterer Vorteil der Füllungsmaterialien auf Amalgam-Basis ist die komfortable Verarbeitung. Das Material ist im frischgemischten Zustand leicht verformbar und passt sich auch in komplizierte Innengeometrien ein. Es kann durch Druckausübung (Stopfen) in die Kavität eingepresst werden. Es wurde bereits versucht, diese "Handling"-Eigenschaften des Amalgams mit feinkörnigen Composite-Massen zu imitieren (DE 44 43 702 A1/US 5,886,064).

Angenehm für den Verarbeiter ist auch das zeitliche Verhalten der Härtung bei Amalgam: Nach dem Anmischen - für gewöhnlich in der Kapsel in einem Mischgerät - ist das Material 2 bis 5 Minuten lang leicht zu verarbeiten und zu stopfen. Dann folgt eine Härtungsphase, die 5 bis 10 Minuten dauert. Das Material ist dann hart genug, um nach einem Polierschritt den Patienten zu entlassen, mit der üblichen Verhaltensmaßregel, 2 Stunden keine feste Nahrung zu sich zu nehmen.

Aufgabe der Erfindung ist es, ein selbsthärtendes Material bereitzustellen, das hinsichtlich dreier Kriterien: des charakteristischen zeitlichen Verlaufs der Härtung, der Verarbeitbarkeit und der Randspaltdichte dem bekannten Amalgam möglichst nahekommt.

Im Vorliegenden wird daher ein Zahnbfüllungsmaterial vorgeschlagen, das im wesentlichen
- A: aus einer organischen selbsthärtenden Komponente und
- B: einer anorganischen Komponente, die eine Abbindereaktion unter Kristallbildung und Expansion eingeht, sowie gegebenenfalls
- C: geeigneten Füllstoffen
besteht, und das den gleichen zeitlichen Härtungsverlauf wie Silberamalgam aufweist.

Das System ist vorteilhaft ein Zweikomponentensystem aus Pulver und Flüssigkeit. Die Abbinde- bzw. Polymerisationsreaktionen treten ein, wenn die Komponenten gemischt werden. Es ist dabei vorteilhaft, das System ähnlich dem Amalgam in Kapseln anzubieten, die in die herkömmlichen Mischer passen.

Die organische selbsthärtende Komponente ist ein an sich bekanntes selbsthärtendes System, z.B. wie in US 4,547,531 oder US 5,688,883 beschrieben.

Die anorganische Komponente ist vorteilhaft ein auf Zementhärtung basierendes System aus Pulver und Wasser, z.B. eine Mischung auf der Basis von Ammoniumphosphat und Magnesiumoxid.

Zweckmäßig werden die Komponenten getrennt als Pulver und Flüssigkeit bereitgestellt. Die Pulverkomponente enthält in der Regel noch inerte Füllstoffe, welche die mechanischen Eigenschaften der Mischung beeinflussen. Vor der Anwendung werden Pulver und Flüssigkeit zu einer Paste angemischt. Die geschieht zweckmäßig in Kapseln, die in einen herkömmlichen Amalgam- oder Zementmischer gegeben werden. Die Mischbewegung führt zur Vereinigung der flüssigen und festen Komponenten, vorteilhaft zu einer Paste. Die Paste kann dann entnommen und verarbeitet werden.

Erfindungswesentlich ist, dass das Härtungsverhalten dem von herkömmlichem Silberamalgam gleichkommt. Damit ist gemeint, dass der Anwender hinsichtlich der Arbeitsweise und des Zeitaufwands der einzelnen Schritte keinen wesentlichen Unterschied feststellt. Die Verarbeitungszeit, in der die Masse noch formbar ist, beträgt bei dem üblichen Silber-Zinn-Amalgam 3 bis 5 Minuten. Dieses Kriterium wird auch vom erfindungsgemäßen Material erfüllt. Dasselbe gilt auch für die Abbindezeit, die im Bereich von 5 bis 10 Minuten liegt. Letztlich ist keine Umstellung im Vergleich zur Herstellung von Amalgamfüllungen nötig. Die organische Zweikomponentenmasse härtet dabei in ähnlicher Zeit, in der auch die Zementhärtung der anorganischen Komponente erfolgt. Die organische Komponente ist selbsthärtend ausgelegt, d.h. es werden Initiatoren wie z.B. thermischen Initiatoren oder Redoxinitiatorsysteme eingesetzt.

Als thermische Initiatoren kommen insbesondere organische Peroxide in Form von Diacylperoxiden, Peroxydicarbonaten, Alkylperestern, Dialkylperoxiden, Perketalen, Ketonperoxiden und Alkylhydroperoxiden in Frage. Konkrete und bevorzugte Beispiele für thermische Initiatoren sind Dibenzoylperoxid, t-Butylperbenzoat und Azobisisobutyronitril. Der oxidierende Teil der Redoxinitiatorsysteme kann z.B. eine Verbindung aus der Gruppe Benzoylperoxid, Laurylperoxid, Benzoin, Benzophenon sowie der alpha-Diketone sein. Bevorzugt ist Benzoylperoxid. Der reduzierende Teil der Redoxinitiatorsysteme entstammt z.B. der Gruppe der tertiären Amine. Bevorzugt davon sind N,N-Dimethyl-para-toluidin und N,N-Dimethyl-sym-xylidin.

Bevorzugt sind die Initiatoren zu 0.3 bis 1.5 Gew.. % in der Mischung enthalten.

Bei der Auswahl der Füller wird die Konsistenz von Amalgam angestrebt. Geeignete Füllstoffe sind sogenannte Mikrofüller, deren Körnung in nm-Bereich liegt, wie etwa hochdisperse Kieselsäure und sogenannte Makrofüller, deren Körnung im Mikrometerbereich liegt, insbesondere körnige Kieselsäure oder gemahlene Dentalgläser. Bei den Gläsern handelt es sich vorzugsweise um Aluminiumsilicatgläser, die mit Barium, Strontium oder seltenen Erden dotiert sein können (DE-PS 24 58 380). Bevorzugt sind fein gemahlene Gläser oder Quarz mit mittleren Teilchengrößen zwischen etwa 1 und 10 Mikrometer sowie hochdisperses SiO₂ mit mittleren Teilchengrößen zwischen etwa 10 und 400 nm.

### Beispiel:

Als geeignete Mischung hat sich folgende erwiesen:
Anorganisches Bindersystem auf Basis Ammoniumphosphat/Magnesiumoxid 25%.
Inerte Füllstoffe 60%.
Organische Zweikomponentenmasse 15%.
Die Masse wird in einem Amalgammischer vermischt und in eine Kavität gefüllt.
Die Verarbeitungszeit bis zur Härtung beträgt 4 bis 5 Minuten.
Die Härtungszeit beträgt 10 bis 13 Minuten.

## Patentansprüche

1. Zahnfüllungsmaterial aus Pulver und Flüssigkeit, im wesentlichen bestehend aus
A einer organischen selbsthärtenden Komponente,
B einer anorganischen Komponente, die eine Abbindereaktion unter Kristallbildung und Expansion eingeht,
C Wasser,
**dadurch gekennzeichnet, dass** es nach Anmischen in einer Verarbeitungszeit von 2 bis 5 Minuten plastisch verformbar bleibt und dann binnen 10 bis 15 Minuten härtet.

2. Zahnfüllungsmaterial nach Anspruch 1, zusätzlich enthaltend
D organische biokompatible Lösungsmittel.

3. Zahnfüllungsmaterial nach Anspruch 1, zusätzlich enthaltend
E geeignete Füllstoffe.

4. Zahnfüllungsmaterial nach einem der Ansprüche 1 bis 3, bei dem die anorganische und/oder die organische Komponente bei der Härtung expandieren.

5. Zahnfüllungsmaterial nach einem der Ansprüche 1 bis 4, bei dem die organische selbsthärtende Komponente ein Zweikomponentensystem aus Pulver und Flüssigkeit ist.

6. Zahnfüllungsmaterial nach Anspruch einem der Ansprüche 1 bis 5, bei dem die anorganische Komponente ein auf Zementhärtung basierendes System aus Pulver und Wasser ist.

7. Zahnfüllungsmaterial nach Anspruch 6, bei dem das Pulver eine Mischung aus Ammoniumphosphat und Magnesiumoxid enthält.

8. Zahnfüllungsmaterial nach einem der Ansprüche 1 bis 7, bei dem die Füllstoffe eine Mischung von gemahlenem Dentalglas und Kieselsäure sind.

9. Zahnfüllungsmaterial nach Anspruch 8, bei dem das Dentalglas Bariumaluiminiumsilikatglas ist.
